# EUROPEAN PATENT APPLICATION

(11) **EP 4 626 016 A1**
(43) Date of publication of application: **01.10.2025**
(21) Application number: 24856110.2
(22) Date of filing: 10.06.2024
(51) Int. Cl.: H04R 1/00, A61N 7/00, G10K 11/30, H04R 1/32, H04R 17/00

(54) **ULTRASONIC VIBRATOR AND ULTRASONIC THERAPEUTIC APPARATUS HAVING SAME**

(30) Priority: 22.08.2023 JP 2023135037
(71) Applicant: Sound Wave Innovation Co., Ltd., Tokyo 103-0026 (JP)
(72) Inventor: YOSHIKAWA Yasuo, Tokyo 103-0026 (JP); KOBAYASHI Kazuto, Toyohashi-shi, Aichi 441-3193 (JP); SHIMOKAWA Hiroaki, Narita-shi, Chiba 286-8686 (JP)
(74) Representative: Bandpay & Greuter
(86) International application number: PCT/JP2024/021057
(87) International publication number: WO 2025/041415

(57) **Abstract**

A transducer body (11) having a radiation surface (11a) that is curved in shape, the transducer body being configured to generate an ultrasound wave, and a transducer cover (12) that covers the radiation surface (11a) of the transducer body (11) and serves as an acoustic matching layer are provided. The radiation surface is of a convex spherical shape, and the transducer cover (12) conforms to the shape of the radiation surface (11a) of the transducer body (11). Thus, an ultrasonic transducer that has high durability and can more stably deliver a sound wave to a deep portion is provided.

## Description

### TECHNICAL FIELD

The present disclosure relates to an ultrasonic transducer whose transducer body that generates ultrasound waves is covered with a transducer cover and an ultrasonic treatment device including the ultrasonic transducer.

### BACKGROUND ART

Conventionally, a device for treating dementia that includes a plurality of ultrasound probes, an ultrasonic transducer arranged in the ultrasound probes to transfer non-converged ultrasonic energy to a brain, and an ultrasound generating device connected to the ultrasound probes has been known (for example, see International Patent Publication No. WO2018/181991).

The device is configured to promote angiogenesis and neurogenesis by applying low-intensity pulsed ultrasound (LIPUS) to a disease site of a brain, a heart, or the like to enhance expression of endothelial nitric oxide synthetase (which will be hereinafter referred to as eNOS) and vascular endothelial growth factor (which will be hereinafter referred to as VEGF).

### SUMMARY OF THE INVENTION

### TECHNICAL PROBLEM

However, in a known ultrasonic transducer, although a sound wave can be applied to the entire brain or the entire heart that is a target of ultrasound application by a single transducer or a plurality of transducers, the sound wave generated from a radiation surface thereof is difficult to reach a deep portion with a constant pressure because of a vibration mode and interference of the sound wave.

Moreover, there is a problem in which, since the radiation surface of the known ultrasonic transducer has a special shape, the known ultrasonic transducer is vulnerable to impact and a protective film provided for water-proof on a surface is easily peeled off due to heat and impact.

In view of the foregoing, the present disclosure has been devised and it is therefore an object of the present disclosure to provide an ultrasonic transducer that has high durability and can more stably deliver a sound wave to a deep portion.

### SOLUTION TO THE PROBLEM

In order to achieve the objective described above, in the present disclosure, a radiation surface of a transducer body is covered with a cover that extends along a curved surface of the transducer body.

Specifically, according to a first aspect of the present disclosure, an ultrasonic transducer includes a transducer body having a radiation surface that is curved in shape, the transducer body being configured to generate an ultrasound wave, and a transducer cover that covers the radiation surface of the transducer body and serves as an acoustic matching layer, and the transducer cover conforms to the shape of the radiation surface of the transducer body.

According to the above-described configuration, since the radiation surface of the transducer body is curved in shape, an applied waveform advances while being diffused. Spread of sound in the transducer cover is determined by Snell's law and, since a material of the transducer cover is normally composed of a solid material, such as a resin molded product or the like, and has a higher sound velocity than that of water or an organism, a refractive index is large and, a result, a range of radiation from the transducer cover is increased, that is, the ultrasound wave is radiated from a wide surface, as compared to a case where the transducer cover is not provided. Thus, constant radiation is enabled. Furthermore, a distance from the transducer body that is a heating body to an organism or the like is ensured by the transducer cover, and therefore, a problem of a surface temperature rise can be reduced.

According to a second aspect of the present disclosure, in the ultrasonic transducer according to the first aspect, the radiation surface of the transducer body is of a convex spherical shape.

Normally, a wave that is radiated from the radiation surface of the transducer body is considered as a collection of point light sources with the radiation surface as a reference and, when the radiation surface is a convex spherical surface, a portion in which sounds from the point light sources interfere with each other to weaken each other or strengthen each other is generated, and a fixed sound field is not eventually generated. However, according to the above-described configuration, the transducer cover covers the radiation surface, and thus, more constant radiation from a wide surface is enabled.

According to a third aspect of the present disclosure, in the ultrasonic transducer according to the first aspect, the transducer cover has a thickness that is constant.

According to the above-described configuration, fine adjustment of the thickness of the cover is not necessary, and manufacturing is facilitated.

According to a fourth aspect of the present disclosure, in the ultrasonic transducer according to the third aspect, the transducer cover has a thickness that is λ/8 or more and 3λ or less and is outside a range of ±λ/40 of each of λ/8, λ/4, and λ/2, λ being a wavelength of a material of the transducer cover.

According to the above-described configuration, a resonance structure is achieved, and by deliberately forming the transducer cover such that the transducer cover has a thickness that is not around a thickness (λ/8, λ/4, and λ/2) that allows maximum transmission, the transducer cover that serves as an acoustic matching layer that reduces variations in radiation of the ultrasonic transducer. This is because, a problem in which, when a small deviation from a thickness with which an amplitude is maximum occurs, radiation energy rapidly changes can be avoided. Considering that, when the transducer cover is too thin, an effect of diffusion due to refraction is reduced, and also, in view of not only acoustic performance but also manufacturability and durability of the transducer cover, the transducer cover that is thicker than λ/8 is preferable. When the transducer cover has a larger thickness than 3λ, attenuation is too large, and it is difficult to actually use the transducer cover.

According to a fifth aspect of the present disclosure, in the ultrasonic transducer according to the first aspect, the transducer cover has a thickness that is not constant but varies.

According to the above-described configuration, spread can be controlled to be proper by causing the thickness of the transducer cover to vary, not to be constant.

According to a sixth aspect of the present disclosure, in the ultrasonic transducer according to the first aspect, the transducer cover is formed of a material having an elastic modulus of 2000 MPa or more and 10000 MPa or less and an acoustic characteristic impedance of 1.5 MPa·s/m or more and 10 MPa·s/m or less.

According to the above-described configuration, the elastic modulus of the material of the transducer cover is kept at a proper level and, since the material has a higher sound velocity than that of water or an organism, a refractive index is large, so that a range of radiation from the transducer cover is further increased.

An ultrasonic treatment device according to a seventh aspect of the present disclosure includes the ultrasonic transducer according to any one of the first to sixth aspects, and a system configured to supply energy to the ultrasonic transducer to generate ultrasonic vibration.

According to the above-described configuration, for example, in the ultrasonic treatment device that can perform treatment of angina pectoris and dementia using expression enhancement of eNOS or VEGF, a constant sound pressure can be caused to reach a deep position and durability of the ultrasonic transducer can be increased.

### ADVANTAGES OF THE INVENTION

As has been described above, according to the present disclosure, the radiation surface of the transducer body that has a curved surface shape is covered by the transducer cover that serves as an acoustic matching layer that extends along the radiation surface, so that the ultrasonic transducer that has high durability and can more stably deliver a sound wave to a deep portion can be achieved.

### BRIEF DESCRIPTION OF THE DRAWINGS

FIG. 1 is a block diagram illustrating an outline of an ultrasonic treatment device including an ultrasonic transducer according to an embodiment of the present disclosure.
FIG. 2 is an enlarged cross-sectional view of the ultrasonic transducer.
FIG. 3A is a cross-sectional view of a transducer body and a transducer cover, illustrating a case where the transducer cover has a thickness that is constant.
FIG. 3B is a cross-sectional view of the transducer body and the transducer cover, illustrating a case where a central portion of the transducer cover has a small thickness.
FIG. 3C is a cross-sectional view of the transducer body and the transducer cover, illustrating a case where the central portion of the transducer cover has a large thickness.
FIG. 4 is a view illustrating simulation results for a sound pressure level without a cover.
FIG. 5A is a view illustrating simulation results for a sound pressure level corresponding to FIG. 3A.
FIG. 5B is a view illustrating simulation results for a sound pressure level corresponding to FIG. 3B.
FIG. 5C is a view illustrating simulation results for a sound pressure level corresponding to FIG. 3C.
FIG. 6A is a view illustrating simulation results without a cover where it is assumed that a wavelength of a cover material is λ.
FIG. 6B is a view illustrating simulation results when the cover material has a thickness that is λ/8 where it is assumed that the wavelength of the cover material is λ.
FIG. 6C is a view illustrating simulation results when the cover material has a thickness that is λ/3.4 where it is assumed that the wavelength of the cover material is λ.
FIG. 7A is a view illustrating simulation results when a cover having a thickness of λ/4 is attached.
FIG. 7B is a view illustrating simulation results when a cover having a thickness of λ/3.4 is attached.

### DESCRIPTION OF EMBODIMENTS

Embodiments of the present disclosure will be described below with reference to the accompanying drawings.

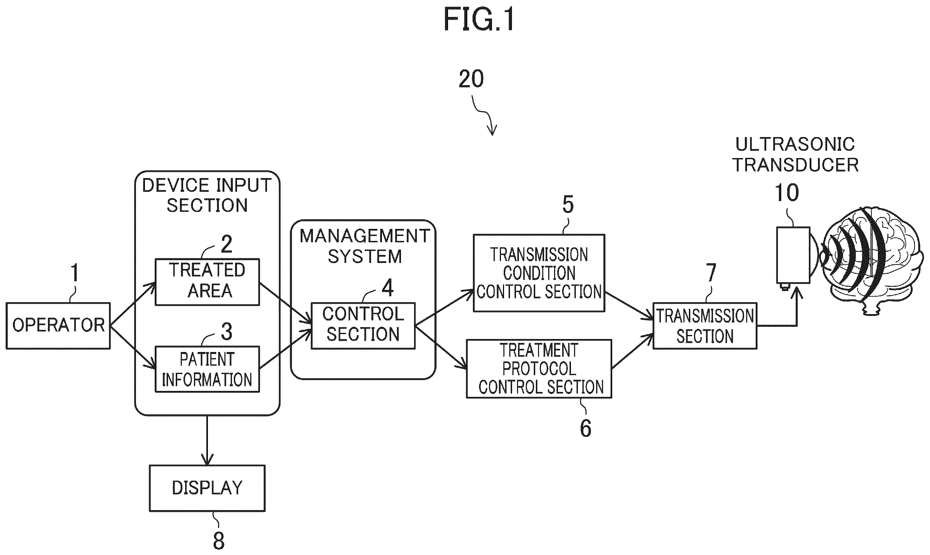

FIG. 1 illustrates an outline of an ultrasonic treatment device 20 including an ultrasonic transducer 10 according to an embodiment of the present disclosure. Although use of the ultrasonic transducer 10 is not particularly limited to what is described below, the ultrasonic treatment device 20 is configured to transmit a frequency in an ultrasonic range and perform radiation of LIPUS to a heart or a brain to perform treatment for angina pectoris, heart failure with preserved ejection fraction (HFpEF), and dementia using expression enhancement of eNOS or VEGF.

To describe the outline of the ultrasonic treatment device 20, in the ultrasonic treatment device 20, for treatment information that has been input by an operator 1, that is, for example, a treated area 2 and patient information 3, a signal is sent by a control section 4 that serves as a protocol management system in the ultrasonic treatment device 20 and a transmission waveform and a treatment protocol are transmitted, after being controlled in a transmission condition control section 5 and a treatment protocol control section 6, respectively, from a transmission section 7 and are radiated to a brain from the treated area 2 (for example, a heard portion) by the ultrasonic transducer 10. The control section 4, the transmission condition control section 5, and the treatment protocol control section 6 are formed of, for example, one or more microcomputers built in the ultrasonic treatment device 20.

When two or more ultrasonic transducers 10 are provided, a signal is branched into a plurality of signals at the transmission section 7 and the signal is radiated to each of the ultrasonic transducers 10. For example, when the ultrasonic transducer 10 is used for a head set, a pair of left and right ultrasonic transducers 10 are needed.

Information of the ultrasonic treatment device 20 is indicated to the operator 1 via a display 8. As for a method for inputting information to the ultrasonic treatment device 20, input of the information is performed by external input via a keyboard, a mouse, or the like, input by a touch operation when the display 8 is a touch display, insertion of an USB memory or the like.

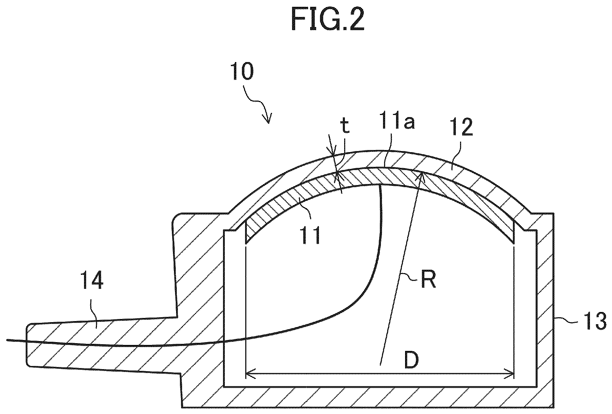

As enlarged and illustrated in FIG. 2, the ultrasonic transducer 10 that is used herein includes a transducer body 11, and the transducer body 11 is a diffusion-type transducer a radiation surface 11a of which has a curved surface shape. Since the radiation surface 11a of the transducer body 11 has a curved surface shape, a radiated waveform advances while being diffused.

As a result, radiation to the entire treated area 2 as a target is enabled. For example, when the target is a brain, a radiation range preferably extends with an angle of 77 degrees or more.

The ultrasonic transducer 10 includes a transducer cover 12 that ensures biological safety and protects the ultrasonic transducer 10 from impact of the transducer body 11 to prevent damage thereto. The ultrasonic transducer 10 includes a transducer housing 13 that covers the transducer body 11 and a transducer cable 14 through which electricity is transmitted to the transducer body 11 at a back side of the transducer cover 12. The transducer housing 13 may be an integrated with the transducer cover 12 as a single body and may be a separate body from the transducer cover 12.

As the transducer body 11, for example, a piezoelectric element, such as a piezo (PZT) element, a homopolymer (PVDF) of vinylidene fluoride (VdF) that is a type of fluororesin, barium titanate (BaTiO₃), relaxor-based piezoelectric single crystal (PIN-PmN-PT) or the like, elements that are formed using a semiconductor process and are called a capacitance-type micromachine ultrasonic transducer (CMUT) and a piezoelectric-type micromachine ultrasonic transducer (PMUT), or the like can be used.

A frequency of an ultrasound wave that is applied to the transducer body 11 is a frequency (the number of cycles) matched with a frequency of the transducer body 11. The frequency is 1 cycle or more and 64 cycles or less and is preferably 24 cycles or more and 40 cycles or less. An average strength within a pulse width (Ispta) is not particularly specified, but is 720 mW/cm² or less and is preferably 150 mW/cm² or less.

The frequency of the transducer body 11 (a frequency near a resonance point or an antiresonance point) is, for example, 100 kHz or more and 10 MHz or less. The transducer body 11 is of a convex spherical shape, a radius R of a spherical surface illustrated in FIG. 2 is 10 mm or more and 30 mm or less, and an opening diameter D of the spherical surface is 10 mm or more and 50 mm or less. A backing formed of air, oil, or a solid material is provided at a back side of the transducer body 11. Note that the backing is a component that is arranged at a back of the transducer body 11, suppresses rearward propagation of the ultrasound wave, and contributes to shortening the pulse width.

As a material of the transducer cover 12, a material having an elastic modulus of 2000 MPa or more and 10000 MPa or less and an acoustic characteristic impedance of 1.5 MPa·s/m or more and 10 MPa·s/m or less is preferable.

Furthermore, when a wavelength of the material of the transducer cover 12 is λ, it is preferable that the transducer cover 12 has a thickness that is λ/8 or more and 3λ or less and is outside a thickness in a range of ±λ/40 of each of λ/8, λ/4, and λ/2.

The transducer cover 12 preferably conforms to the shape of the radiation surface of the transducer body 11.

Examples of the material of the transducer cover 12 include a synthetic resin material, such as polyphenylene ether (PPE), polybutylene terephthalate (PBT), acrylonitrile/butadiene/styrene (ABS), low-density polyethylene (LDPE), polystyrene (PS), NORYL (registered trademark), Valox, Pebax, or the like, a composite material of any one of carbon, graphite, metal, or the like and resin, or the like.

An adhesive that adheres a back surface of the transducer cover 12 and the radiation surface 11a of the transducer body 11 is preferably, for example, an epoxy adhesive, a silicone adhesive, or the like.

As for waterproof performance of the transducer cover 12, the material of the transducer cover 12 itself may have the waterproof performance and the waterproof performance may be achieved by applying thin waterproof coating of parylene (a series of polymers that is obtained from paraxylene) in a range that the waterproof coating does not affect sound.

As for insulation property, insulation is needed in a portion of the transducer cover 12 that is a patient contact portion. For example, since the transducer body 11 has a ground layer (GND) on a surface, when the thickness of the transducer cover 12 is less than 0.4 mm or when a withstand voltage of the transducer cover 12 is less than 1500 V, it is necessary to separately provide an insulating layer. For example, examples of the insulating layer include polyvinyl chloride coating (PVC), fluorine coating (ETFE), polyethylene coating, nylon coating, epoxy coating, PPS/PEEK coating, or the like.

### - Working Examples -

Normally, a wave that is radiated from the radiation surface 11a of the transducer body 11 is considered as a collection of point light sources with the radiation surface 11a as a reference. With the convex spherical shape of the transducer body 11, because of the shape, a portion in which sounds from the point light sources interfere with each other to weaken each other or strengthen each other is generated, and a fixed sound field is not eventually generated.

In this embodiment, in order to solve this problem, the transducer cover 12 is attached to the radiation surface 11a of the transducer body 11.

Due to an effect of the transducer cover 12 described above, radiation at a fixed sound pressure level is enabled in a wide range. Although spread of sound in the transducer cover 12 is determined by Snell's law (law of refraction), resin that is the material of the transducer cover 12 normally has a higher sound velocity than that of water or an organism (the sound velocity of water is 1480 m/sec at 20 °C), and therefore, a refractive index is large. As a result, a range of radiation from the transducer cover 12 is wide, that is, radiation is made from a wide surface, as compared to a case where the transducer cover 12 is not provided. Thus, more constant radiation is enabled.

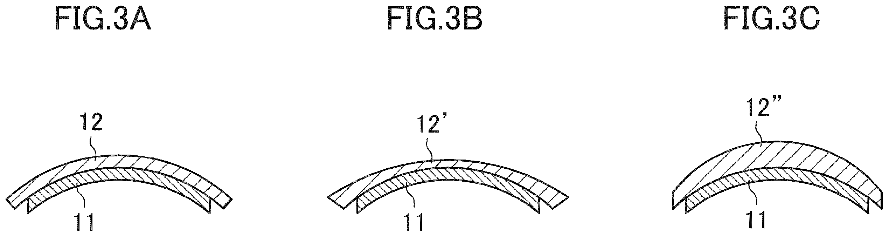

In each of FIG. 3A to FIG. 3C, an example of the shapes of the transducer body 11 and the transducer cover 12 is illustrated. The transducer cover 12 of FIG. 3A has a uniform constant thickness, in a transducer cover 12' of FIG. 3B, a central portion is thin, and in a transducer cover 12" of FIG. 3C, a central portion is thick.

Herein, when the wavelength of the material of the transducer cover 12 is λ, a thickness of the transducer cover 12 of FIG. 3A is t = λ/3.4, a thickness of the transducer cover 12' of FIG. 3B is t = λ/4 at center and t = λ/2 at both end portions, and a thickness of the transducer cover 12" of FIG. 3C is t = λ × 3/4 at center and t = λ/2 at both end portions.

Results of sound analysis will be described below. The sound analysis was conducted at a frequency of 500 kHz with an overall dimension set to 80 mm, and the sound pressure level dB is indicated in different colors by a linear scale.

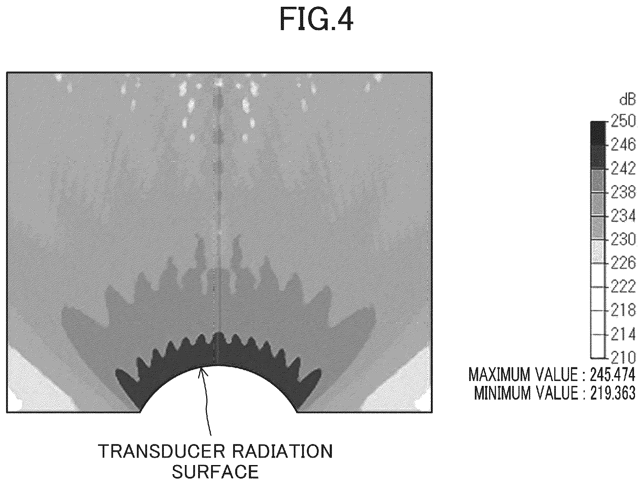

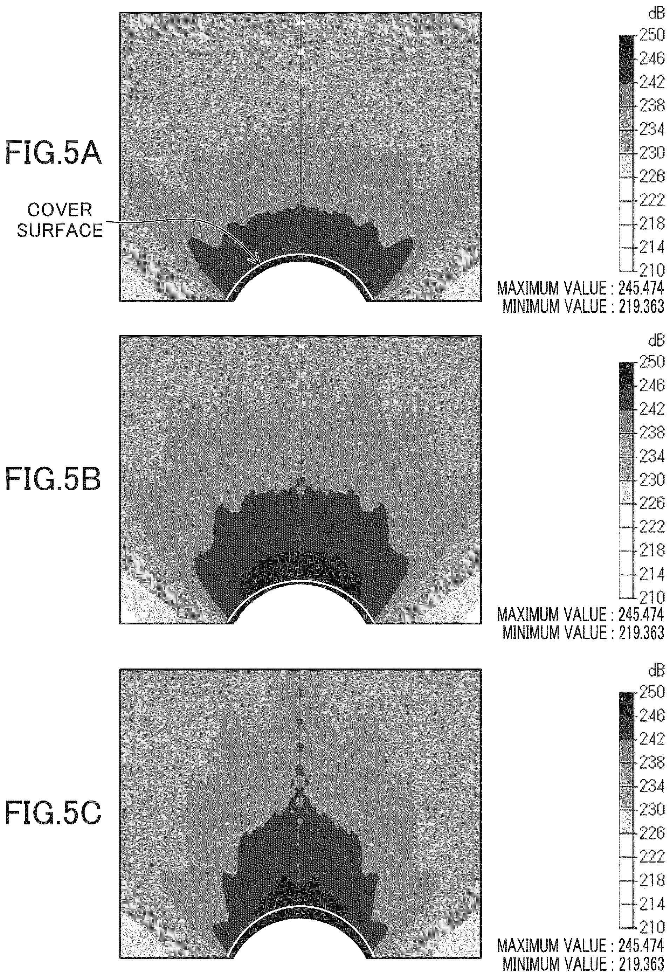

A simulation result for the sound pressure level when a cover is not provided is illustrated in FIG. 4, and results of simulations for the sound pressure levels corresponding to FIG. 3A to FIG. 3C are illustrated in FIG. 5A to FIG. 5C, respectively. It can be seen that, as compared to a case illustrated in FIG. 4 where the transducer cover 12 was not provided, in each of cases illustrated in FIG. 5A to FIG. 5C where the transducer cover 12, the transducer cover 12', and the transducer cover 12" were provided, a sound pressure uniformly spreads deeper and wider.

In order to perform constant radiation, the thickness of the transducer cover 12 is needed to be a certain value or more. This is because, when the transducer cover 12 is too thin, an effect of diffusion due to refraction is reduced.

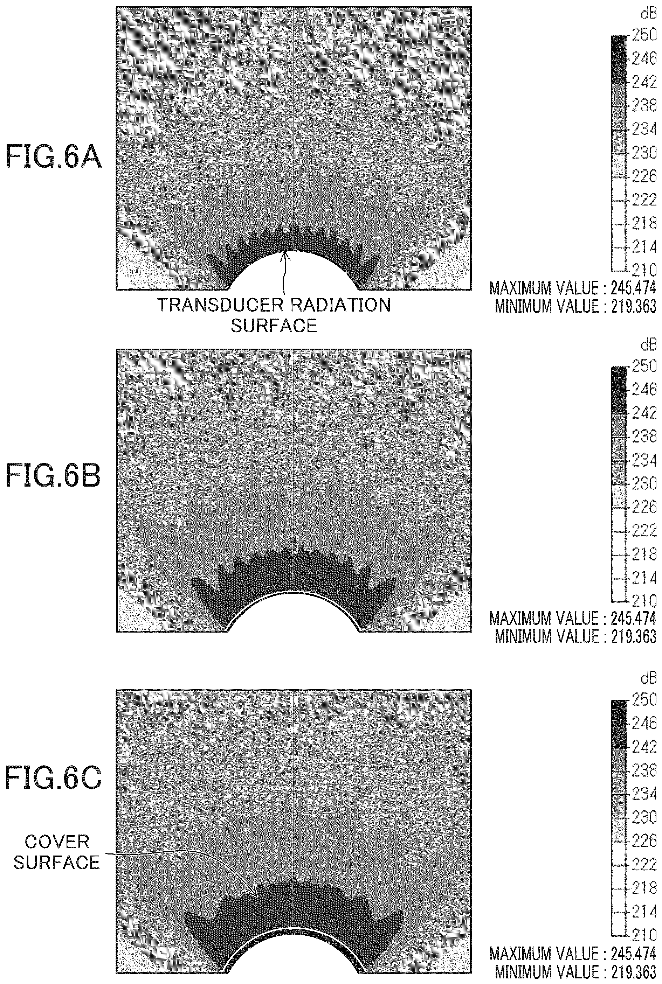

As results of the sound analysis, a result for a case where a cover was not provided, a result for a case where a thickness of a cover material was λ/8, and a result for a case where a thickness of a cover material was λ/3.4 in a condition where the wavelength of the material of the transducer cover 12 was λ are illustrated in FIG. 6A, FIG. 6B, and FIG. 6C, respectively. It can be seen that, although there was an influence of attenuation of the transducer cover 12, the sound pressure was uniform to a deeper position in the case where the transducer cover 12 was provided. Moreover, it was found that, in the case of FIG. 6C where the thickness was λ/3.4, that is, where the thickness of the transducer cover 12 was larger than that in the case of FIG. 6B where the thickness was λ/8, a sound level at a high level could be applied to a deeper position. Based on the forgoing, it was found that a cover that was thicker than that in the case where the wavelength was λ/8 was more preferable.

When it is assumed that the sound velocity is 2200 m/s and the transmission frequency is 500 kHz, λ/8 = 0.55 mm. In view of not only acoustic performance but also manufacturability and durability of the transducer cover 12, the transducer cover 12 having a larger thickness than λ/8 is preferable.

Moreover, it can be considered not only that spread varies depending on difference in the sound velocity but also that the transducer cover 12 also plays a role as an acoustic matching layer. When the material used for the transducer cover 12 is a piezo element, an impedance is about 30 MPa·s/m. When the sound velocity of the transducer cover 12 is 200 m/s and a density of a resin material is 1.2 g/cm³, the impedance is 2.6 MPa·s/m. In contrast, an impedance of water is 1.44 MPa·s/m, and the impedance of the resin material is between respective values of PZT and water. Radiation can be more efficiently performed by reducing reflection at a boundary surface and an unnecessary vibration mode by the transducer cover 12, and therefore, it is considered that the transducer cover 12 contributes to constant radiation.

In a normal ultrasonic transducer, an acoustic matching layer is attached, in order to increase efficiency thereof, the acoustic matching layer is designed to have a thickness (for example, λ/2, λ/4, λ/8, or the like) obtained by dividing the wavelength λ by 2 × n (n is an integer). This is because, by designing the acoustic matching layer into this thickness, a radiation surface of the matching layer is an opening end and an amplitude is increased to maximum.

However, there arises a problem in which, when a small deviation from this thickness occurs, radiation energy rapidly changes. This shows that, when a thickness, a frequency, sound velocity of a cover, or the like changes due to variations in manufacturing, variations in devices occur.

In this embodiment, by deliberately forming the transducer cover 12 with a thickness other than a thickness that allows maximum transmission, the transducer cover 12 is set to be the acoustic matching layer that reduces variations in radiation in the ultrasonic transducer 10.

As a method for setting the transducer cover 12 in the above-described manner, the transducer cover 12 having a thickness that is different from a value obtained by dividing the wavelength λ of the material of the transducer cover 12 by 2 × n (n is an integer), that is, for example, a thickness of λ/2, λ/4, or λ/8, by λ/40 or more is attached.

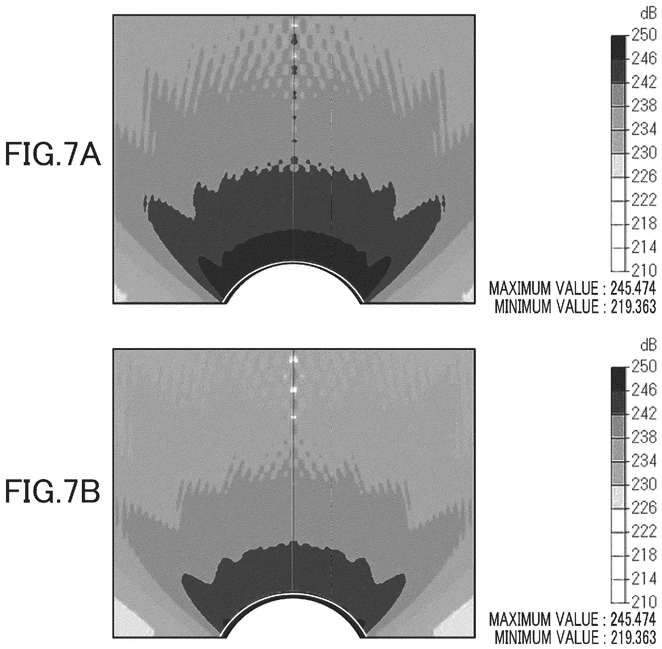

As an example, results of simulations where a cover having a thickness of λ/4 and a cover having a thickness of λ/3.4 were attached are illustrated in FIG. 7A and FIG. 7B, respectively. Although a difference in thickness is about λ/23, which is small, it can be seen that there is a large difference in sound pressure level. This means that, since the cover having a thickness of λ/4 has a resonance structure, energy can be radiated with very high efficiency with this thickness, and also means that the sound pressure level is rapidly changed by changing the thickness. The transducer cover 12 also has a feature that, when the thickness is λ/3.4, a local sound difference is small, as compared to when the thickness is λ /4.

There is also a problem in which the ultrasonic transducer 10 generates heat during vibration. The ultrasonic transducer 10 contacts a patient, and there is probability that an injury, such as a burn or the like, is caused by the heat generation. Although an upper limit for a surface temperature rise is determined by a protocol, a distance from a heating body (the transducer body 11) to an organism is ensured by the transducer cover 12, and therefore, a problem of the surface temperature rise can be also reduced by the transducer cover 12.

Sound spread can be controlled to be proper by varying the thickness of the transducer cover 12, not to be constant.

By deliberately setting the transducer cover 12 to a thickness other than a half-wavelength, a 1/4 wavelength, and a 1/8 wavelength that is a design policy of a normal acoustic matching layer, an influence of difference in thickness in a manufacturing step can be reduced as much as possible.

The transducer body 11 has a feature that the transducer body 11 is fragile to impact or the like because of the shape thereof, but a property with respect to impact can be increased by sticking any one of the transducer cover 12, transducer cover 12', and the transducer cover 12" each of which is formed of a resin material having a high elastic modulus and a large thickness thereto.

The ultrasonic treatment device 20 of the present disclosure is a device that can output an ultrasound wave widely to a target site and can radiate a predetermined transmission waveform to a brain and a heart, so that expression of eNOS and VEGF can be enhanced, angiogenesis and neurogenesis can be promoted, and treatment for angina pectoris, heart failure with preserved ejection fraction (HFpEF), or dementia can be performed. In the ultrasonic transducer 10 having a convex curved surface, although, because of the special shape thereof, a problem arises in which the pressure is not constant in a deeper position due to a vibration mode and interference of the sound wave, the shape of the transducer cover 12 of this embodiment is in accordance with the shape of the radiation surface 11a and a resin that can be used as an acoustic matching layer is stuck to the radiation surface 11a, so that an unnecessary vibration mode can be suppressed, an influence of interference can be reduced, and, even with the same output, constant radiation to a deeper position can be performed.

### (Other Embodiments)

The above-described embodiment of the present disclosure may be configured in the following manner.

That is, in the above-described embodiment, an example where the ultrasonic transducer 10 is used for the ultrasonic treatment device 20 has been described, but the ultrasonic transducer 10 may be used for an ultrasonic washing machine, an ultrasonic humidifier, an ultrasonic dispersing and emulsifying machine, or the like. Furthermore, the ultrasonic transducer 10 can be used for a fish finder, an ultrasonic diagnosis device, an ultrasonic speed meter, an ultrasonic level meter, or the like.

In the above-described embodiment, the radiation surface 11a is of an approximately perfect circular convex spherical shape when viewed from top, but the radiation surface 11a may be formed into an elliptical shape, an oblong circular shape, a rounded rectangular shape, a rounded squire, or the like, when viewed from top, having a convex curved surface shape.

Note that the above-described embodiments are merely preferable examples by nature and are not intended to be particularly limiting the present disclosure, application of the present disclosure, and the scope of use.

### DESCRIPTION OF REFERENCE CHARACTERS

- 1: operator
- 2: treated area
- 3: patient information
- 4: control section
- 5: transmission condition control section
- 6: treatment protocol control section
- 7: transmission section
- 8: display
- 10: ultrasonic oscillator
- 11: oscillator body
- 11a: radiation surface
- 12, 12', 12": oscillator cover
- 13: oscillator housing
- 14: oscillator cable
- 20: ultrasonic treatment device

## Claims

1. An ultrasonic transducer, comprising:
a transducer body having a radiation surface that is curved in shape, the transducer body being configured to generate an ultrasound wave; and
a transducer cover that covers the radiation surface of the transducer body and serves as an acoustic matching layer,
wherein
the transducer cover conforms to the shape of the radiation surface of the transducer body.

2. The ultrasonic transducer of claim 1, wherein
the radiation surface of the transducer body is of a convex spherical shape.

3. The ultrasonic transducer of claim 1, wherein
the transducer cover has a thickness that is constant.

4. The ultrasonic transducer of claim 3, wherein
the transducer cover has a thickness that is λ/8 or more and 3λ or less and is outside a range of ±λ/40 of each of λ/8, λ/4, and λ/2, λ being a wavelength of a material of the transducer cover.

5. The ultrasonic transducer of claim 1, wherein
the transducer cover has a thickness that is not constant but varies.

6. The ultrasonic transducer of claim 1, wherein
the transducer cover is formed of a material having an elastic modulus of 2000 MPa or more and 10000 MPa or less and an acoustic characteristic impedance of 1.5 MPa·s/m or more and 10 MPa·s/m or less.

7. An ultrasonic treatment device comprising:
the ultrasonic transducer of any one of claims 1 to 6; and
a system configured to supply energy to the ultrasonic transducer to generate ultrasonic vibration.
